# EUROPEAN PATENT SPECIFICATION

(11) **EP 3 351 163 B1**
(45) Date of publication and mention of the grant of the patent: **04.03.2020**
(21) Application number: 17152541.3
(22) Date of filing: 20.01.2017
(51) Int. Cl.: A61B 5/00, H04W 56/00, H04W 84/18

(54) **METHOD FOR PROVIDING SYNCHRONIZATION BETWEEN A PLURALITY OF WIRELESS BODY SENSORS AND METHOD FOR OPERATING A SYNCHRONIZED NETWORK OF WIRELESS BODY SENSORS**
VERFAHREN ZUR BEREITSTELLUNG VON SYNCHRONISATION ZWISCHEN EINER VIELZAHL VON DRAHTLOSEN KÖRPERSENSOREN UND VERFAHREN ZUM BETREIBEN EINES SYNCHRONISIERTEN NETZWERKS VON DRAHTLOSEN KÖRPERSENSOREN
PROCÉDÉ DE SYNCHRONISATION ENTRE UNE PLURALITÉ DE CAPTEURS CORPORELS SANS FIL ET PROCÉDÉ DE FONCTIONNEMENT D'UN RÉSEAU SYNCHRONISÉ DE CAPTEURS CORPORELS SANS FIL

(43) Date of publication of application: 25.07.2018
(73) Proprietor: Byteflies NV, 2600 Antwerpen (BE)
(72) Inventor: Danneels, Hans, B-2800 Mechelen (BE); De Clercq, Hans, B-9040 Sint-Amandsberg (BE); Safavi, Hossein, Schererville, IN 46375 (US); Vandendriessche, Benjamin, B-9052 Zwijnaarde (BE)
(74) Representative: IPLodge bvba

(56) References cited:
- EP-A1- 2 117 180
- KR-A- 20140 111 418
- JOHAN PLOMP ET AL: "Considerations for Synchronization in Body Area Networks for Human Activity Monitoring", INTERNATIONAL JOURNAL OF WIRELESS INFORMATION NETWORKS, KLUWER ACADEMIC PUBLISHERS-PLENUM PUBLISHERS, NE, vol. 18, no. 4, 3 May 2011 (2011-05-03), pages 280-294, XP019981304, ISSN: 1572-8129, DOI: 10.1007/S10776-011-0136-2

## Description

### Field of the Invention

The present invention pertains to the field of wireless body sensors, in particular to methods for providing synchronization in networks of such wireless body sensors. The present invention also pertains to a method for operating a synchronized network of wireless body sensors, a wireless body sensor, a scheduler, and a monitoring system.

### Background

The problem of providing timing information to a plurality of network nodes with a view of synchronizing their processes is well known. In this area, European patent application publication no. EP 0722233 A2 in the name of Hewlett Packard Co. discloses a data communication network comprising a local clock within a node of the network which may be synchronized and syntonized by any node in the network. Each node contains a time packet detector that detects and recognizes timing data packets and produces a recognition signal. Each node has a time server that includes the local clock. The time server records the time of the recognition signal. The recorded time is used for correcting the local clocks of the various nodes in the network. A transfer device such as a gateway, a bridge or a router may include a time server and a time packet detector to correct for the transit time of a time packet through such transfer device. The time packet detector is connected at the point of final encoding for transmission or recovery of the clock and data. Other examples of prior art are KR 2014 0111418, EP 2 117 180 and the article "Considerations for Synchronization in Body Area Networks for human activity monitoring by J. Plomp et al.

In a wireless setting, one or more of the nodes in the network may be unable to receive a specific timestamp for several reasons, e.g. because the nodes are in wireless standby in order to conserve power, because the nodes determine that they are still in sync with the signal and therefore turn off their radio receiver, or because the unreliable nature of wireless signaling causes a reception error.

To avoid these synchronization failures, the synchronization signal is typically sent repeatedly (like a heartbeat) to ensure that the potentially numerous nodes within the network have the best chances of receiving the relevant synchronization signals.

It is a disadvantage of these existing systems that at the master node(s), energy is continuously being used to transmit the heartbeat signal. This makes the known synchronization methods unsuitable for battery-operated wireless devices with a limited energy autonomy, such as those used in body area sensor networks.

### Summary

According to an aspect of the present invention, there is provided a method for providing synchronization between a plurality of wireless body sensors, the method comprising: establishing a time schedule designating different individual sensors of the plurality of wireless body sensors as a master node for respective consecutive periods of time; operating the plurality of wireless body sensors whereby the different individual sensors broadcast a periodically repeated synchronization signal during the respective consecutive periods of time; analyzing an energy consumption and performance pattern of the plurality of wireless body sensors under the time schedule; and updating the time schedule on the basis of the energy consumption and performance pattern.

In known master/slave synchronization methods, the master or masters remain(s) unchanged for the duration of operation. The present invention is based on the insight of the inventors that energy use by the network nodes can be optimized by distributing the responsibility of the synchronization master over multiple nodes. This leads to reduced energy storage requirements, hence smaller batteries, and hence smaller nodes.

The synchronization system of the present invention is particularly advantageous for battery-constrained multi-sensor networks. This includes in particular sensor networks used in medical applications that require accurate comparison of the timing of events (features) derived from multiple waveforms.

In an embodiment of the method according to the present invention, the operating, the analyzing, and the updating are performed iteratively.

It is an advantage of this embodiment, that the time schedule is periodically updated to remain optimized even as the usage characteristics of individual sensors change over time.

In an embodiment of the method according to the present invention, the updating comprises optimizing the time schedule to obtain a maximal value of an expected battery life of the sensor predicted to run out of battery first.

It is an advantage of employing this maximin-type strategy that the overall autonomous lifetime of the sensor swarm as whole - which is limited by the first sensor to fail - is maximized.

According to an aspect of the present invention, there is provided method for operating a synchronized network of wireless body sensors, the method comprising at each of the wireless body sensors: storing a time schedule designating different individual sensors of the plurality of wireless body sensors as a master node for respective consecutive periods of time assuming and relinquishing a master node role in accordance with the stored time schedule; while the master node role is assumed, broadcasting a periodically repeated synchronization signal; and while the master node role is not assumed, receiving the periodically repeated synchronization signal from the master node.

It is an advantage of this embodiment that the power usage across the swarm of sensors can be optimized over longer periods of time, by judiciously handing over the responsibility for mastering the synchronization from one node to the next.

According to an aspect of the present invention, there is provided a wireless body sensor, comprising: a memory for storing a time schedule designating said wireless body sensor as a master node for one or more periods of time; and a processor configured to: assume and relinquish a master node role in accordance with said stored time schedule; while said master node role is assumed, broadcast a periodically repeated synchronization signal; and while said master node role is not assumed, receive said periodically repeated synchronization signal from another node acting as master node.

According to an aspect of the present invention, there is provided a scheduler comprising a processor configured to: establish a time schedule designating different individual sensors of a plurality of wireless body sensors as a master node for respective consecutive periods of time; analyze an energy consumption and performance pattern of said plurality of wireless body sensors under said time schedule; update said time schedule on the basis of said energy consumption and performance pattern; and transmit said updated time schedule to said plurality of wireless body sensors.

According to an aspect of the present invention, there is provided a monitoring system comprising a plurality of wireless body sensors as described above and a scheduler as described above.

The technical effects and advantages of embodiments of the scheduler, the wireless body sensor, and the monitoring system according to the present invention correspond, *mutatis mutandis,* to those of the corresponding embodiments of the methods according to the present invention.

### Brief Description of the Figures

These and other features and advantages of embodiments of the present invention will now be described in more detail with reference to the attached drawings in which:
- Figure 1 provides a flow chart of an embodiment of the method for providing synchronization between a plurality of wireless body sensors according to the present invention;
- Figure 2 provides a flow chart of an embodiment of the method for operating a synchronized network of wireless body sensors according to the present invention; and
- Figures 3-8 illustrate exemplary use cases of embodiments of the present invention.

### Description of Embodiments

Reliable analysis of waveform features derived from multiple physiologic signals requires accurate signal synchronization. Evaluation of the inter-feature timing for two or more sensor signals can generate synergistic and physiologically relevant information in comparison to the same sensor signals studied in isolation. The resulting multimodal waveform features can be used to develop novel physiologic biomarkers and generate cohort and population-level insights into various disease processes. The methodology for accurate synchronization of multiple battery-constrained sensor nodes disclosed herein will facilitate the development of new medical applications, and the development of novel analysis techniques for synchronized bio-signals.

In the method according to the present invention, the sensors periodically connect to a scheduling node (e.g. this can be the docking station at the end of the night when charging or through wireless communication with a scheduling device). Optionally, one of the sensor nodes themselves may also be the scheduler that identifies the working master schedule. Then, the nodes/sensors indicated in the schedule will take the role of the master synchronization node as assigned. This intelligent scheduling improves synchronization performance and power consumption. Consider that the network is in fact a series of battery powered wireless nodes. Hence, the constraints of the battery place an inherent limitation on the master node which will have higher power consumption than the other nodes. In order to share this synchronization responsibility, we can intelligently schedule various nodes to be the synchronization master at different times.

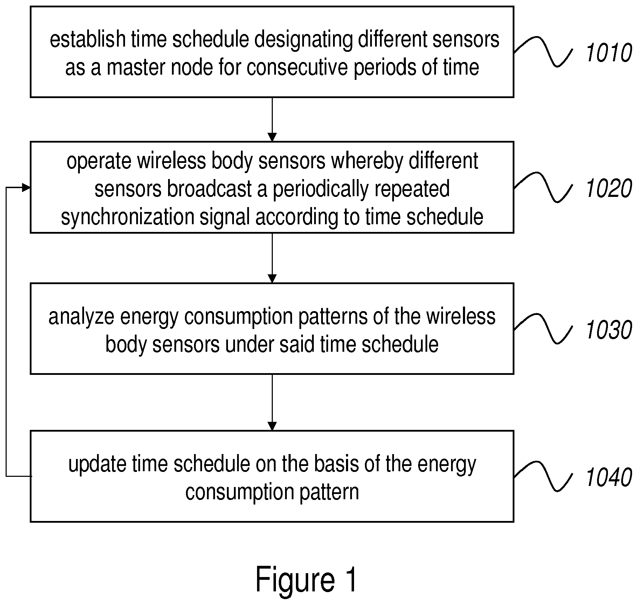

To that end, we propose a system where the sensors periodically connect to a scheduling node as described above. The steps involved are illustrated in an exemplary and non-limiting way in the flow chart of Figure 1.

In a first step **1010,** an initial time schedule is established, designating different individual sensors of the plurality of wireless body sensors as a master node for respective consecutive periods of time. If no detailed power consumption data of the sensors is known at the initial stage, the initial time schedule may be established in a trivial way, e.g. by dividing the total required time period to be spanned (e.g. one 24-hour period) in equal parts over randomly selected sensors, or in an order based on the sensors' serial number or the like. If an analysis of the power consumption profile of the sensors has already taken place, the initial step **1010** may substantively be the same as the updating step **1040** described below.

In a second step **1020,** the plurality of wireless body sensors is operated, i.e. sensor data is gathered according to the purpose of each sensor. To this end, the initially established schedule may be transmitted to the sensors by a scheduler, the sensors storing this schedule in a memory. To maximize the autonomy of the wireless sensor network, the operation preferably starts with all sensors having fully charged batteries. A periodically repeated synchronization signal (a so-called "heartbeat signal") is transmitted at all times by a master node to the other sensors. Thus, different individual sensors broadcast the periodically repeated synchronization signal during the respective consecutive periods of time in which they are designated as the master node, while the other sensors receive the synchronization signal to maintain synchronization.

In a third step **1030,** an energy consumption pattern of the plurality of wireless body sensors under the time schedule is analyzed.

This analysis **1030** may occur subsequently to the operating **1020.** It may comprise reading out the remaining battery levels of all the sensors. If the initial battery level of the sensors is known, the employed time schedule is known, and the remaining battery level of all the sensors is known, it is possible to determine the amount of energy consumed under the employed schedule by each of the sensors. The energy consumption may be measured in relative terms, i.e. as a percentage of the total energy storage capacity of each individual sensor.

Alternatively or additionally, more detailed statistics may be gathered at each of the sensor nodes may be used. For example, each sensor node may record its own typical power consumption during the day, the signal strength of the received synchronization signals from each master, the typical drift it experiences from the reference clocks, whether some nodes are configured with larger batteries, etc.

The analysis **1030** may also occur on an ongoing basis during said operating **1020,** and trigger a time schedule update **1040** as described below when necessary.

In a fourth step **1040,** the time schedule is updated on the basis of the energy consumption pattern. For example, if the analysis **1030** reveals that some sensors have undergone during the operation **1020** a low amount of relative energy consumption while other sensors have undergone during the operation **1020** a high amount of relative energy consumption, it may be more optimal to assign the master node role to sensors of the former group during longer periods of time in the updated time schedule. For example, signal strength may also be considered so as to select master nodes that can be overheard by most of the other nodes (e.g. perhaps a sensor located on an extremity of the body will be less suitable as a master).

As symbolized by the arrow returning from the fourth step **1040** to the second step **1020,** the operating **1020,** the analyzing **1030,** and the updating **1040** are performed iteratively.

This iteration may for example occur on a diurnal basis, e.g. when the sensors are docked in a charging/scheduling station every evening, which receives the operational information from the sensors and transmits the updated schedule to the sensors. If the usage characteristics of individual sensors change over time, the regular updating of the time schedules ensures that an optimal time schedule is used at all times.

Alternatively or additionally, the iteration may be triggered during operation, for example if the observed energy consumption patterns reveal that the intended time schedule cannot be maintained due to an unexpected excessive power consumption of specific sensors. In that case, the sensors may communicate with a node adapted to impose a new time schedule on the wireless body sensors; this may be one of the wireless body sensors, a docking station, or a dedicated node. The sensors may also employ a distributed decision-making protocol by which they "elect" a new time schedule.

Preferably, the updating **1040** comprises optimizing the time schedule to obtain a maximal value of an expected battery life of the sensor predicted to run out of battery first. The network of sensors is most valuable when all sensors are active; thus, it is advantageous to maximize the minimal battery life expected to occur in the entire group.

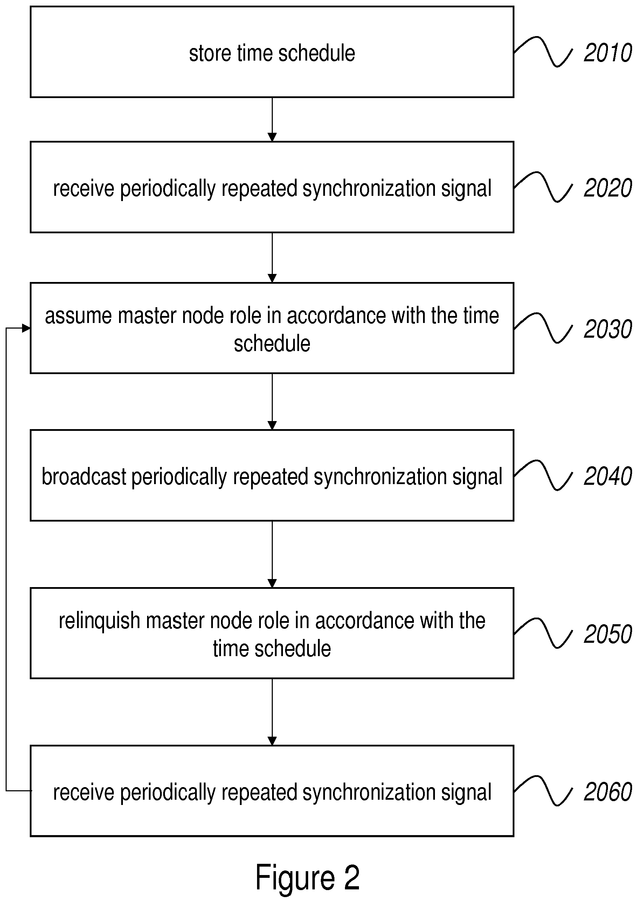

The steps as performed by the individual wireless body sensor nodes are illustrated in an exemplary and non-limitative manner in Figure 2.

Each wireless body sensor stores **2010** a time schedule designating different individual sensors of the plurality of wireless body sensors as a master node for respective consecutive periods of time. This storing **2010** may take place when the sensors are docked to the scheduler, as part of a regular charging and updating routine. In operation, the wireless body sensor assumes **2030** and relinquish **2050** a master node role in accordance with the stored time schedule. While the master node role is assumed - i.e. between said assuming **2030** and said relinquishing **2050** - the wireless body sensor acts as the master node by broadcasting **2040** the periodically repeated synchronization signal. While said master node role is not assumed - i.e. outside said assuming **2030** and said relinquishing **2050** - the wireless body sensor acts as a slave node by receiving **2020, 2060** the periodically repeated synchronization signal from a different sensor that acts as the master node. During the entire operation, the wireless body sensor may gather and store, in addition to its sensing data, relevant operational information such as power consumption patterns, signal strength levels, and the like, which may be used by the scheduler as explained in the context of Figure 1.

The time schedule may provide that a given sensor node assumes and relinquishes the master node role more than once, as symbolized by the arrow returning from step **2060** to step **2030.**

The sensor network synchronized according to the present invention may be used to obtain additional insights in the features being sensed. Using signal processing techniques, relevant physiologic features may be extracted from each synchronized waveform, thereby generating a synchronized feature matrix. Any combination of pairwise distance comparisons can then be performed across sensor-feature instances to generate multimodal bio-signals. For instance:
1. Synchronization of cardiac contraction and peripheral blood flow: high-accuracy correlation of a ventricular contraction and the arrival of the resulting blood pulse in the peripheral circulation can be used for more precise cardiovascular characterization (e.g. pulse travel time, blood pressure trends, ...) than either sensor used in isolation.
2. Synchronization of multiple peripheral blood flow sensors: high-accuracy correlation of multiple peripheral blood flow signals can be used for more precise hemodynamic characterization (e.g. vessel compliance, vascular resistance, ...) than either sensor used in isolation.
3. Synchronization of cardiac contraction and respiration: high-accuracy correlation of the timing of a respiratory phase transition and its associated preceding and trailing ventricular contractions can be used for evaluation of cardiorespiratory coupling (e.g. respiratory sinus arrhythmia, cardioventilatory coupling, ...).
4. Synchronization of multiple motion sensors: high accuracy correlation of positional information of the limbs and torso can be used to evaluate posture, gait dynamics and activity.
5. Synchronization of a cardiovascular feature and brain activity: high-accuracy synchronization of event-related potentials derived from brain activity measurements and one or more cardiovascular features can be used to analyze the brain-heart axis.

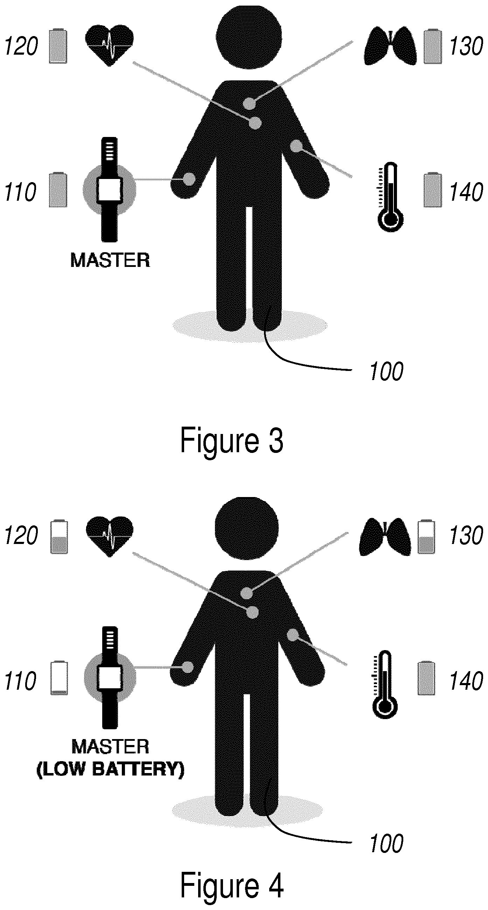

An exemplary use case will now be described, without loss of generality, with reference to Figures 3-8.

A user (patient) **100** with a need for continuous cardiovascular monitoring wears a body area network comprising:
1. A chest patch with sensors for electrocardiogram (ECG) **120** and respiration **130** detection;
2. A wrist-worn bracelet **110** with sensors for pulse plethysmography (PPG) and activity detection;
3. A patch with sensors **140** for temperature and electrodermal activity (EDA) detection.

These and other devices can be combined in configurations that interfere least with the user's routine. At the start of a day, while the user **100** is in his or her residence, the nodes **110-140** in the body area network transmit diagnostics information from the previous day (such as battery discharge rate, activity, and received signal strength) to a designated scheduling device **200** (i.e. a docking station).

The docking station **200** uses this information to intelligently select a synchronization master.

Throughout the day, the nodes **110-140** may take turns serving as the synchronization master based on the schedule provided by the docking station **200** or, if needed, elect a new synchronization master based on power consumption patterns (either via the docking station or by communication between the devices themselves).

In the present example, as illustrated in Figure 3, the PPG wrist sensor **110** was originally elected as the master node.

The user **100** may be more active than usual and the PPG sensor's **100** battery may be depleted faster than the other sensors, as illustrated in Figure 4.

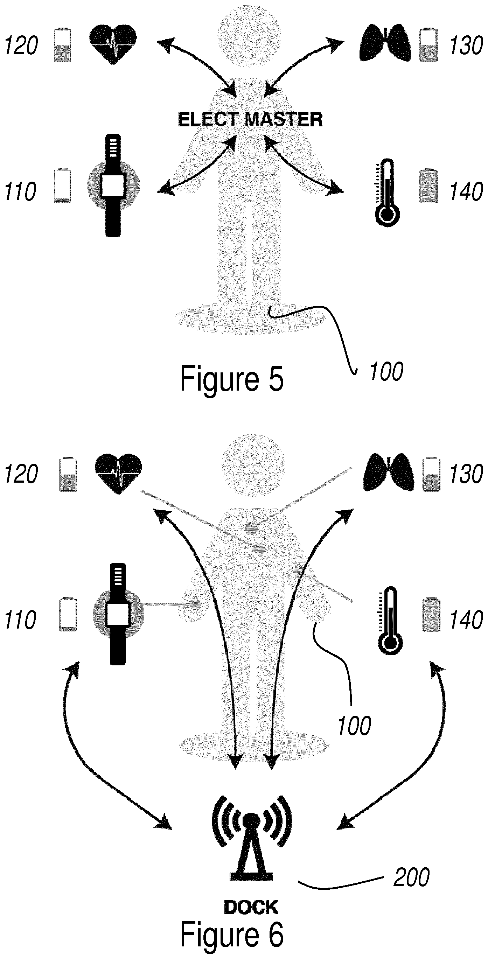

The devices **110-140** communicate this information to each other and can opt to elect a new master, as illustrated in Figure 5.

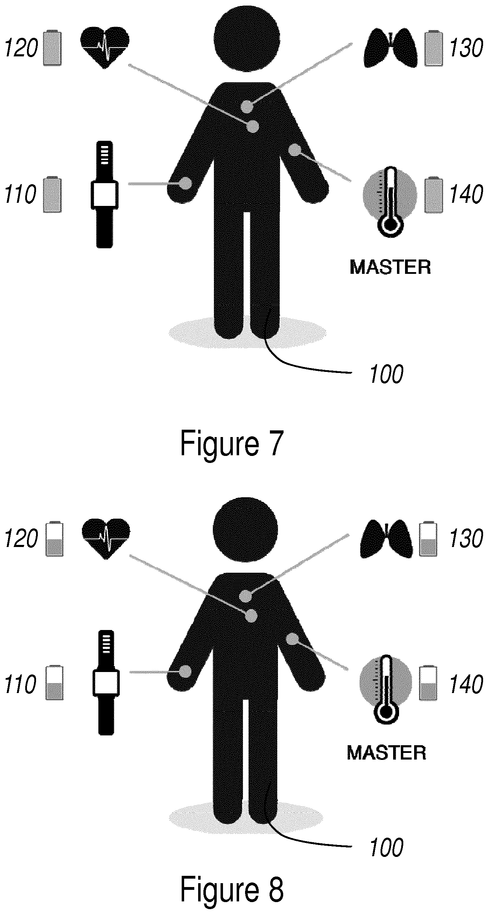

Alternatively, if the dock **200** is nearby, it can assign a new master node as well (Figure 6), e.g. the temperature sensor **140** (Figure 7), resulting in more optimally distributed power consumption (Figure 8).

In addition, the sensors **110-140** continuously measure their respective signals. After uploading, the data is processed and relevant features are extracted. Due to the high accuracy of the time synchronization of the signals, multimodal features can be extracted. Together, the collection of bio-signals can be used to track changes in the patient's cardiovascular health status, and eventually generate a comprehensive picture of the user's physiologic phenotype.

While the invention has been described with reference to a limited number of concrete embodiments, this was done to illustrate and not to limit the invention the scope of which is to be determined by referring to the enclosed claims.

## Claims

1. A method for providing synchronization between a plurality of wireless body sensors, the method comprising:
- establishing (1010) a time schedule designating different individual sensors of said plurality of wireless body sensors as a master node for respective consecutive periods of time;
- operating (1020) said plurality of wireless body sensors whereby said different individual sensors broadcast a periodically repeated synchronization signal during said respective consecutive periods of time;
- analyzing (1030) an energy consumption and performance pattern of said plurality of wireless body sensors under said time schedule; and
- updating (1040) said time schedule on the basis of said energy consumption and performance pattern.

2. The method according to claim 1, wherein said operating (1020), said analyzing (1030), and said updating (1040) are performed iteratively.

3. The method according to claim 1 or claim 2, wherein said updating (1040) comprises optimizing said time schedule to obtain a maximal value of an expected battery life of the sensor predicted to run out of battery first.

4. A method for operating a synchronized network of wireless body sensors, the method comprising at each of said wireless body sensors:
- storing (2010) a time schedule designating different individual sensors of said plurality of wireless body sensors as a master node for respective consecutive periods of time
- assuming (2030) and relinquishing (2050) a master node role in accordance with said stored time schedule;
- while said master node role is assumed, broadcasting (2040) a periodically repeated synchronization signal; and
- while said master node role is not assumed, receiving (2020, 2060) said periodically repeated synchronization signal from said master node.

5. A wireless body sensor (110, 120, 130, 140), comprising:
- a memory for storing (2010) a time schedule designating said wireless body sensor (110, 120, 130, 140) as a master node for one or more periods of time; and
- a processor configured to:
∘ assume (2030) and relinquish (2050) a master node role in accordance with said stored time schedule;
∘ while said master node role is assumed, broadcast (2040) a periodically repeated synchronization signal; and
∘ while said master node role is not assumed, receive (2020, 2060) said periodically repeated synchronization signal from another node acting as master node.

6. A scheduler (200) comprising a processor configured to:
- establish (1010) a time schedule designating different individual sensors of a plurality of wireless body sensors (110, 120, 130, 140) as a master node for respective consecutive periods of time;
- analyze (1030) an energy consumption and performance pattern of said plurality of wireless body sensors (110, 120, 130, 140) under said time schedule;
- update (1040) said time schedule on the basis of said energy consumption and performance pattern; and
- transmit said updated time schedule to said plurality of wireless body sensors (110, 120, 130, 140).

7. A monitoring system comprising a plurality of wireless body sensors (110, 120, 130, 140) according to claim 5 and a scheduler (200) according to claim 6.

## Patentansprüche

1. Verfahren zum Bereitstellen einer Synchronisation zwischen mehreren drahtlosen Körpersensoren, wobei das Verfahren Folgendes umfasst:
- Erstellen (1010) eines Zeitplans, der unterschiedliche einzelne Sensoren der mehreren drahtlosen Körpersensoren für jeweilige aufeinanderfolgende Zeiträume als einen Hauptknoten bestimmt,
- Betreiben (1020) der mehreren drahtlosen Körpersensoren, wobei die unterschiedlichen einzelnen Sensoren während der jeweiligen aufeinanderfolgenden Zeiträume ein periodisch wiederholtes Synchronisationssignal rundsenden,
- Analysieren (1030) eines Energieverbrauchs- und Leistungsmusters der mehreren drahtlosen Körpersensoren unter dem Zeitplan und
- Aktualisieren (1040) des Zeitplans auf der Grundlage des Energieverbrauchs- und Leistungsmusters.

2. Verfahren nach Anspruch 1, wobei das Betreiben (1020), das Analysieren (1030) und das Aktualisieren (1040) iterativ durchgeführt werden.

3. Verfahren nach Anspruch 1 oder Anspruch 2, wobei das Aktualisieren (1040) das Optimieren des Zeitplans umfasst, um einen maximalen Wert einer zu erwartenden Batterielebensdauer des Sensors zu erreichen, von dem vorhergesagt wird, dass er zuerst die Batterie entleert.

4. Verfahren zum Betreiben eines synchronisierten Netzes von drahtlosen Körpersensoren, wobei das Verfahren an jedem der drahtlosen Körpersensoren Folgendes umfasst:
- Speichern (2010) eines Zeitplans, der unterschiedliche einzelne Sensoren der mehreren drahtlosen Körpersensoren für jeweilige aufeinanderfolgende Zeiträume als einen Hauptknoten bestimmt,
- Annehmen (2030) und Abgeben (2050) einer Hauptknotenrolle in Übereinstimmung mit dem gespeicherten Zeitplan,
- während die Hauptknotenrolle angenommen wird, Rundsenden (2040) eines periodisch wiederholten Synchronisationssignals, und
- während die Hauptknotenrolle nicht angenommen wird, Empfangen (2020, 2060) des periodisch wiederholten Synchronisationssignals von dem Hauptknoten.

5. Drahtloser Körpersensor (110, 120, 130, 140), der Folgendes umfasst:
- einen Speicher zum Speichern (2010) eines Zeitplans, der den drahtlosen Körpersensor (110, 120, 130, 140) für einen oder mehrere Zeiträume als einen Hauptknoten bestimmt, und
- einen Prozessor, der zu Folgendem konfiguriert ist:
∘ eine Hauptknotenrolle in Übereinstimmung mit dem gespeicherten Zeitplan anzunehmen (2030) und abzugeben (2050),
∘ während die Hauptknotenrolle angenommen wird, ein periodisch wiederholtes Synchronisationssignal rundzusenden (2040), und
∘ während die Hauptknotenrolle nicht angenommen wird, das periodisch wiederholte Synchronisationssignal von einem anderen Knoten, der als der Hauptknoten agiert, zu empfangen (2020, 2060).

6. Scheduler (200), das einen Prozessor umfasst, der zu Folgendem konfiguriert ist:
- einen Zeitplan zu erstellen (1010), der unterschiedliche einzelne Sensoren von mehreren drahtlosen Körpersensoren (110, 120, 130, 140) für jeweilige aufeinanderfolgende Zeiträume als einen Hauptknoten bestimmt,
- ein Energieverbrauchs- und Leistungsmuster der mehreren drahtlosen Körpersensoren (110, 120, 130, 140) unter dem Zeitplan zu analysieren (1030),
- den Zeitplan auf der Grundlage des Energieverbrauchs- und Leistungsmusters zu aktualisieren (1040) und
- den aktualisierten Zeitplan an die mehreren drahtlosen Körpersensoren (110, 120, 130, 140) zu übermitteln.

7. Überwachungssystem, das mehrere drahtlose Körpersensoren (110, 120, 130, 140) nach Anspruch 5 und ein Scheduler (200) nach Anspruch 6 umfasst.

## Revendications

1. Procédé pour fournir une synchronisation entre une pluralité de capteurs corporels sans fil, le procédé comprenant :
- l'établissement (1010) d'une planification temporelle désignant différents capteurs individuels de ladite pluralité de capteurs corporels sans fil en tant que nœud maître pendant des périodes de temps consécutives respectives ;
- la mise en œuvre (1020) de ladite pluralité de capteurs corporels sans fil de sorte que lesdits différents capteurs individuels diffusent un signal de synchronisation répété de façon périodique pendant lesdites périodes de temps consécutives respectives ;
- l'analyse (1030) d'un modèle de consommation d'énergie et de performance de ladite pluralité de capteurs corporels sans fil conformément à ladite planification temporelle ; et
- la mise à jour (1040) de ladite planification temporelle sur la base dudit modèle de consommation d'énergie et de performance.

2. Procédé selon la revendication 1, dans lequel ladite mise en œuvre (1020), ladite analyse (1030) et ladite mise à jour (1040) sont effectuées de manière itérative.

3. Procédé selon la revendication 1 ou la revendication 2, dans lequel ladite mise à jour (1040) comprend l'optimisation de ladite planification temporelle pour obtenir une valeur maximale d'une autonomie de batterie attendue du capteur prévu pour être à court de batterie en premier.

4. Procédé pour mettre en œuvre un réseau synchronisé de capteurs corporels sans fil, le procédé comprenant au niveau de chacun desdits capteurs corporels sans fil :
- le stockage (2010) d'une planification temporelle désignant différents capteurs individuels de ladite pluralité de capteurs corporels sans fil en tant que nœud maître pendant des périodes de temps consécutives respectives ;
- le fait de prendre (2030) et de renoncer à (2050) un rôle de nœud maître selon ladite planification temporelle stockée ;
- tandis que ledit rôle de nœud maître est pris, la diffusion (2040) d'un signal de synchronisation répété de façon périodique ; et
- tandis que ledit rôle de nœud maître n'est pas pris, la réception (2020, 2060) dudit signal de synchronisation répété de façon périodique en provenance dudit nœud maître.

5. Capteur corporel sans fil (110, 120, 130, 140), comprenant :
- une mémoire pour stocker (2010) une planification temporelle désignant ledit capteur corporel sans fil (110, 120, 130, 140) en tant que nœud maître pendant une ou plusieurs périodes de temps ; et
- un processeur configuré pour :
-- prendre (2030) et renoncer à (2050) un rôle de nœud maître selon ladite planification temporelle stockée ;
-- tandis que ledit rôle de nœud maître est pris, la diffusion (2040) d'un signal de synchronisation répété de façon périodique ; et
-- tandis que ledit rôle de nœud de maître n'est pas pris, la réception (2020, 2060) dudit signal de synchronisation répété de façon périodique en provenance d'un autre nœud agissant en tant que nœud maître.

6. Planificateur (200) comprenant un processeur configuré pour :
- établir (1010) une planification temporelle désignant différents capteurs individuels d'une pluralité de capteurs corporels sans fil (110, 120, 130, 140) en tant que nœud maître pendant des périodes de temps consécutives respectives ;
- analyser (1030) un modèle de consommation d'énergie et de performance de ladite pluralité de capteurs corporels sans fil (110, 120, 130, 140) conformément à ladite planification temporelle ;
- mettre à jour (1040) ladite planification temporelle sur la base dudit modèle de consommation d'énergie et de performance ; et
- transmettre ladite planification temporelle mise à jour à ladite pluralité de capteurs corporels sans fil (110, 120, 130, 140).

7. Système de surveillance comprenant une pluralité de capteurs corporels sans fil (110, 120, 130, 140) selon la revendication 5 et un planificateur (200) selon la revendication 6.
